# EUROPÄISCHE PATENTSCHRIFT

(11) **EP 0 930 022 B1**
(45) Veröffentlichungstag und Bekanntmachung des Hinweises auf die Patenterteilung: **17.09.2003**
(21) Anmeldenummer: 98122311.8
(22) Anmeldetag: 24.11.1998
(51) Int. Cl.: A23L 2/58, A61K 7/00, A61K 47/06, A23L 1/275, A61K 7/48

(54) **Verwendung von Carotinoid-Aggregaten als Färbemittel**
Use of carotinoid aggregates as colourants
Utilisation d' agrégats de caroténoides comme colorants

(30) Priorität: 21.01.1998 DE 19802134
(43) Veröffentlichungstag der Anmeldung: 21.07.1999
(73) Patentinhaber: BASF AKTIENGESELLSCHAFT, 67056 Ludwigshafen (DE)
(72) Erfinder: Lüddecke, Erik Dr., 67112 Mutterstadt (DE); Auweter, Helmut Dr., 67117 Limburgerhof (DE); Schweikert, Loni Dr., 67122 Altrip (DE)

(56) Entgegenhaltungen:
- SALARES ET AL.: "Excited state (Exciton) interactions in polyene aggregates" JOURNAL OF RAMAN SPECTROSCOPY, Bd. 6, Nr. 6, 1977, Seiten 282-288, XP001012441
- PILL-SOON SONG ET AL.: "On the photoreceptor pigment for phototropism..." PHOTOCHEMISTRY AND PHOTOBIOLOGY, Bd. 19, 1974, Seiten 435-441, XP002940538

## Beschreibung

Die Erfindung betrifft die Verwendung von Carotinoid-Aggregaten als Färbemittel.

Carotinoide sind eine in der Natur weit verbreitete Farbstoffklasse, die bereits in vielfältiger Form zum Färben von Lebensmitteln, Kosmetika und Non-Food-Artikeln eingesetzt werden. Die Anwendungsmöglichkeiten sind allerdings dadurch stark eingeschränkt, daß die Stoffgruppe der Carotinoide generell sehr licht- und sauerstoffempfindlich ist. In der Praxis müssen daher mit Carotinoiden gefärbte Zubereitungen beispielsweise durch Entgasen oder durch Aufbewahrung in lichtundurchlässigen Gefässen vor Licht und Sauerstoff geschützt werden.

Es war daher die Aufgabe, stabile Carotinoid-Formen als Färbemittel bereitzustellen, die die oben genannten Nachteile nicht aufweisen.

Diese Aufgabe wurde erfindungsgemäß gelöst durch die Verwendung von Carotinoid-Aggregaten als Färbemittel.

Die Aggregation von Carotinoiden ist ein in der Literatur bereits bekanntes und in zahlreichen Publikationen beschriebenes Phänomen [P. Song, T. A. Moore, Photochemistry and Photobiology, 19, 435-441 (1974); A. V. Ruban, P. Horton, A. J. Young, J. Photochem. Photobiol. B: Biol., 21, 229-234 (1993); V. R. Salares, N. M. Young, P. R. Carey, H. J. Bernstein, Journal of Raman Spectroscopy, 6(6), 282-288 (1977)].

Carotinoid-Aggregate können beispielsweise dadurch erzeugt werden, daß man eine Lösung eines Carotinoids in einem wassermischbaren, organischen Lösungsmittel wie z.B. Isopropanol, Ethanol, Aceton oder Tetrahydrofuran mit Wasser vermischt.

So können, wie in der oben genannten Literatur beschrieben, bei Wahl der richtigen Mengenverhältnisse von Wasser und organischem Lösungsmittel entweder sogenannte H- oder J-Aggregate erzeugt werden.

Unter H-Aggregaten versteht man eine "kartenspielähnliche" Stapelung der Polyenketten (card-stack aggregate), die sich im UV/Vis-Spektrum durch das Auftreten einer neuen, im Vergleich zur Absorption der monomer vorliegenden Formen hypsochrom verschobenen Bande im Bereich zwischen 320 und 400 nm charakterisieren läßt. J-Aggregate dagegen stellen eine lineare Kopf-Schwanz Verknüpfung (head-tail aggregate) der Polyene dar, die eine bathochrome Verschiebung der UV-Absorption bewirkt.

Diese Aggregate sind somit im Farbton von dem monomer gelösten Carotinoid verschieden und können sowohl visuell an dieser Farbverschiebung als auch UV/Vis-spektroskopisch erkannt werden.

Es wurde nun überraschenderweise gefunden, daß diese Carotinoid-Aggregate bei Bestrahlung mit Licht, beispielsweise mit einer Xenonlampe, eine wesentlich höhere Lichtstabilität aufweisen als die in monomerer Form vorliegenden Carotinoide.

Aufgrund dieser ausgezeichneten Photostabilität eignen sich die Carotinoid-Aggregate hervorragend zum Färben von Lebensmitteln, kosmetischen und pharmazeutischen Zubereitungen sowie von Non-Food-Objekten, insbesondere von solchen Zubereitungen, bei denen der Farbstoff dem Licht ausgesetzt ist.

Als Färbemittel werden von den eingangs genannten Carotinoid-Aggregaten bevorzugt die H- und/oder J-Aggregate, besonders bevorzugt die J-Aggregate verwendet.

Die Carotinoide, die in Form von Aggregaten als Färbemittel eingesetzt werden können, sind die bekannten, natürlichen oder synthetischen Vertreter dieser Klasse, wie z.B. β-Carotin,-Lycopin, Bixin, Zeaxanthin, Cryptoxanthin, Citranaxanthin, Lutein, Canthaxanthin, Astaxanthin. Besonders bevorzugt werden die mittlerweile auch technisch gut zugänglichen Vertreter wie β-Carotin, Astaxanthin oder Lycopin, insbesondere Astaxanthin und/oder Lycopin verwendet.

Bei der erfindungsgemäßen Verwendung der Carotinoid-Aggregate als Färbemittel können diese allein oder vorteilhafterweise in Gegenwart von Schutzkolloiden eingesetzt werden.

Als Schutzkolloide werden beispielsweise Gelatine, Fischgelatine, Stärke, Dextrin, Pflanzenproteine, Pektin, Gummi-Arabikum, Kasein, Kaseinat oder Mischungen davon verwendet. Es können aber auch Polyvinylalkohol, Polyvinylpyrrolidon, Methylcellulose, Carboxymethylcellulose, Hydroxypropylcellulose und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen.

Zur Erhöhung der mechanischen Stabilität der Carotinoid-Formulierung kann es zweckmäßig sein, dem Kolloid einen Weichmacher zuzusetzen, wie Zucker oder Zuckeralkohole, z.B. Saccharose, Glucose, Lactose, Invertzucker, Sorbit, Mannit oder Glycerin.

Die Mengen an Schutzkolloid, Weichmacher und Carotinoid-Aggregat werden im allgemeinen so gewählt, daß ein Endprodukt erhalten wird, das zwischen 0,1 und 100 ppm, bevorzugt zwischen 0,5 und 50 ppm, besonders bevorzugt 1 bis 20 ppm Carotinoid, bevorzugt Astaxanthin oder Lycopin, 20 bis 200 ppm eines Schutzkolloids, 20 bis 200 ppm eines Weichmachers, alle ppm-Angaben bezogen auf die Gesamtmasse der fertigen Zubereitung, sowie gegebenenfalls geringe Mengen eines Stabilisators enthält.

Zur Erhöhung der Stabilität der Carotinoide gegen oxidativen Abbau ist es vorteilhaft, Stabilisatoren wie α-Tocopherol, t-Butyl-hydroxy-toluol, t-Butylhydroxyanisol, Ascorbinsäure oder Ethoxyquin zuzusetzen.

Als Emulgatoren können beispielsweise Ascorbylpalmitat, Polyglycerin-Fettsäureester, Sorbitan-Fettsäureester, Propylenglycol-Fettsäureester oder Lecithin in einer Konzentration von 0 bis 200 Gew.%, vorzugsweise 10 bis 150 Gew.%, besonders bevorzugt 15 bis 80 Gew.%, bezogen auf das Carotinoid, verwendet werden.

Unter Umständen kann es auch vorteilhaft sein, zusätzlich zum Carotinoid-Aggregat ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.%, vorzugsweise 10 bis 300 Gew.%, besonders bevorzugt 20 bis 100 Gew.%, bezogen auf das Carotinoid, zu verwenden.

Gegenstand der vorliegenden Erfindung sind auch Lebensmittel, die Carotinoid-Aggregate, insbesondere Aggregate von Astaxanthin und/-oder Lycopin in Mengen von 0,1 bis 100 ppm, bevorzugt 0,5 bis 50 ppm, besonders bevorzugt 1 bis 20 ppm (bezogen auf die Gesamtmenge der Zubereitung) enthalten.

Bei den Lebensmitteln kann es-sich u.a. um Milchprodukte, Fette, wie z.B. Margarine sowie bevorzugt um Getränke, beispielsweise um "Soft-Drinks" handeln.

Es kann sich bei den gefärbten Getränken sowohl um transparente als auch um trübe, d.h. lichtundurchlässige Zubereitungen handeln. Bevorzugt ist die Verwendung der Carotinoid-Aggregate zur Färbung transparenter Getränkezubereitungen. Die erfindungsgemäßen Carotinoid-Aggregate lassen sich dabei sowohl in Form einer Emulsion oder einer Suspension, als Trockenpulver oder Solubilisat in das Getränk einarbeiten.

Bei der Verwendung der Carotinoid-Aggregate in Lebensmitteln können vorher die nicht für Lebensmittel zugelassenen organischen Lösungsmittel, wie beispielsweise Aceton oder THF, schonend abdestilliert werden, ohne daß es zu einer Änderung der Aggregatform kommt.

Gegenstand der vorliegenden Erfindung sind weiterhin kosmetische und pharmazeutische Zubereitungen, die 0,1 bis 100 ppm, bevorzugt 0,5 bis 50 ppm, besonders bevorzugt 1 bis 20 ppm Carotinoid-Aggregate, bezogen auf die Gesamtmenge der kosmetischen und pharmazeutischen Zubereitung enthalten.

Diese kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche kosmetischen oder pharmazeutischen Präparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, zusätzliche Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als zusätzlich zu den Carotinoid-Aggregaten verwendete Farbstoffe können die für kosmetische Zwecke geeigneten und zugelassenen Substanzen verwendet werden, wie sie beispielsweise in der Publikation "Kosmetische Färbemittel" der Farbstoffkoimmission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Gesamtmenge der Zubereitung - betragen. Die Herstellung der Zubereitung kann in an sich bekannter Weise, d.h. beispielsweise durch Heiß-, Kalt-, Heiß-Heiß/Kalt- bzw. PIT-Emulgierung erfolgen.

Bevorzugte kosmetische oder pharmazeutische Zubereitungen sind beispielsweise transparente Formulierungen in Form durchsichtiger Emulsionen, Solubilisate, Gele oder alkoholisch-wäßriger Lotionen.

In den nachfolgenden Beispielen wird die erfindungsgemäße Verwendung der Carotinoid-Aggregate näher erläutert.

### Beispiel 1

Von einer molekularen Lösung von 50 mg Astaxanthin pro Liter Aceton wurden 5 ml mit 95 ml eines Gemisches aus 70 ml Wasser und 30 ml Aceton bei Raumtemperatur vermischt. Innerhalb von ca. 30 Minuten bildeten sich J-Aggregate, die durch einen Farbumschlag von orange nach rosa direkt sichtbar wurden. Bei 10 minütiger Bestrahlung dieser Aggregat-Lösung in einer Bestrahlungsapparatur (Suntest®, Fa. Heraeus) war keine Farbänderung zu beobachten. Wurde eine Vergleichslösung von molekular gelöstem Astaxanthin (2,5 ppm Astaxanthin in Aceton) unter gleichen Bedingungen bestrahlt, so war die Farbe nach 10 Minuten vollständig verschwunden.

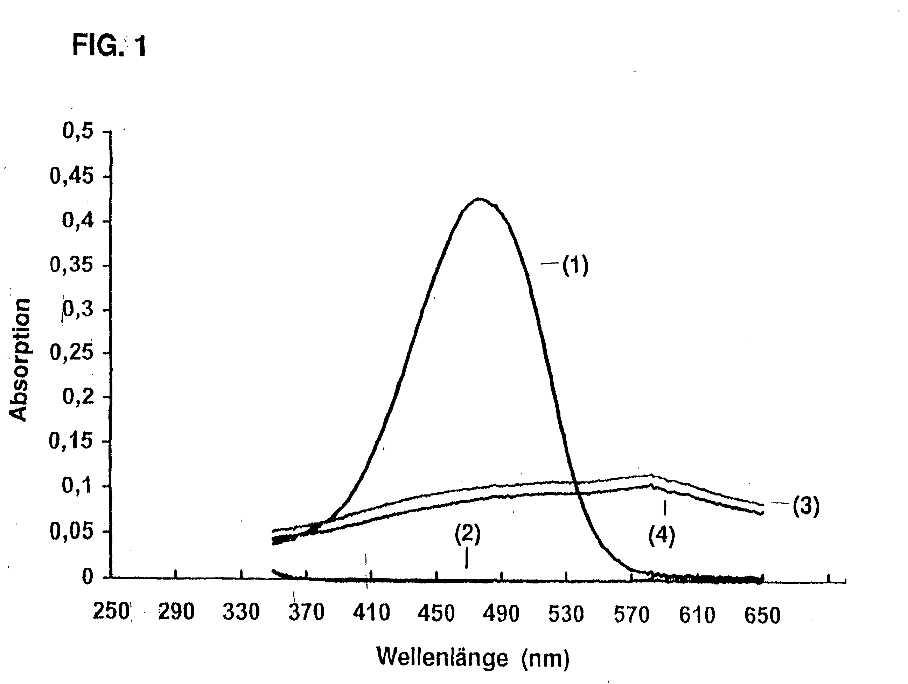

Anhand der vor und nach der Bestrahlung gemessenen UV/Vis-Spektren konnte die erhöhte Lichtstabilität der J-Aggregate von Astaxanthin gegenüber der monomeren Form verdeutlicht werden [siehe FIG. 1: (1) Astaxanthin Monomerform, vor der Bestrahlung; (2) Monomerform, nach der Bestrahlung; (3) Astaxanthin J-Aggregat, vor der Bestrahlung; (4) J-Aggregat, nach der Bestrahlung].

### Beispiel 2

Von einer molekularen Lösung von 50 mg Lycopin pro Liter Isopropanol wurden 5 ml mit 95 ml eines Gemisches aus 30 ml Wasser und 70 ml Isopropanol vermischt. Innerhalb von Sekunden bildeten sich H-Aggregate, die durch eine Farbverschiebung von orange nach gelb-orange direkt sichtbar wurden. Bei 10 minütiger Bestrahlung dieser Aggregat-Lösung in einer Bestrahlungsapparatur (Suntest®, Fa. Heraeus) war nur eine geringe Farbänderung zu beobachten. Die Bestrahlung einer Vergleichslösung von molekular gelöstem Lycopin (2,5 ppm Lycopin in Isopropanol) unter gleichen Bedingungen führte zu einer stärkeren Abnahme der Farbintensität. Durch Verdünnen und Erwärmen konnten die bestrahlten Lösungen in den molekular gelösten Zustand überführt und quantitativ analysiert werden. Das in der Lösung in Aggregatform vorliegende Lycopin war um den Faktor 1,5 stabiler als Lycopin in monomerer Form.

## Patentansprüche

1. Verwendung von Carotinoid-Aggregaten als Färbemittel.

2. Verwendung von Carotinoid-Aggregaten nach Anspruch 1, **dadurch gekennzeichnet, daß** es sich bei den Carotinoid-Aggregaten um H- und/oder J-Aggregate handelt.

3. Verwendung von Carotinoid-Aggregaten nach Anspruch 1, **dadurch gekennzeichnet, daß** es sich bei den Carotinoid-Aggregaten um J-Aggregate handelt.

4. Verwendung von Carotinoid-Aggregaten in der H- und/oder J-Form zum Färben von Lebensmitteln, kosmetischen und pharmazeutischen Zubereitungen.

5. Verwendung von Carotinoid-Aggregaten nach Anspruch 1 bis 4, **dadurch gekennzeichnet, daß** das Carotinoid Astaxanthin oder Lycopin ist.

6. Getränke, enthaltend Carotinoid-Aggregate.

7. Getränke nach Anspruch 6, **dadurch gekennzeichnet, daß** sie transparent sind und als Carotinoide Astaxanthin und/oder Lycopin enthalten.

8. Getränke nach den Ansprüchen 6 und 7 mit einem Carotinoid-Gehalt von 0,1 bis 100 ppm, bezogen auf die Gesamtmenge der Zubereitungen.

9. Kosmetische oder pharmazeutische Zubereitungen, enthaltend Carotinoid-Aggregate.

10. Kosmetische oder pharmazeutische Zubereitungen nach Anspruch 9, **dadurch gekennzeichnet, daß** sie als Carotinoide Astaxanthin und/oder Lycopin enthalten.

11. Kosmetische oder pharmazeutische Zubereitungen nach den Ansprüchen 9 und 10, **dadurch gekennzeichnet, daß** es sich dabei um transparente Zubereitungen handelt.

12. Kosmetische oder pharmazeutische Zubereitungen nach den Ansprüchen 9 bis 11 mit einem Carotinoid-Gehalt von 0,1 bis 100 ppm, bezogen auf die Gesamtmenge der Zubereitungen.

## Claims

1. The use of carotenoid aggregates as colorants.

2. The use of claim 1, wherefor the carotenoid aggregates are H-and/or J-aggregates.

3. The use of claim 1, wherefor the carotenoid aggregates are J-aggregates.

4. The use of carotenoid aggregates in the H- and/or J-form for coloring foods, cosmetic preparations and pharmaceutical preparations.

5. The use of carotenoid aggregates as claimed in any of claims 1 to 4, wherefor the carotenoid is astaxanthin or lycopene.

6. Drinks comprising carotenoid aggregates.

7. Transparent drinks as claimed in claim 6 comprising astaxanthin and/or lycopene as carotenoids.

8. Drinks as claimed in either of claims 6 and 7 having a carotenoid content of from 0.1 to 100 ppm, based on the total amount of drink.

9. Cosmetic or pharmaceutical preparations comprising carotenoid aggregates.

10. Cosmetic or pharmaceutical preparations as claimed in claim 9 comprising astaxanthin and/or lycopene as carotenoids.

11. Transparent cosmetic or pharmaceutical preparations as claimed in either of claims 9 and 10.

12. Cosmetic or pharmaceutical preparations as claimed in any of claims 9 to 11 having a carotenoid content of from 0.1 to 100 ppm, based on the total amount of preparation.

## Revendications

1. Utilisation d'agrégats de caroténoïdes comme colorants.

2. Utilisation d'agrégats de caroténoïdes selon la revendication 1, **caractérisée par le fait qu'**il s'agit pour les agrégats de caroténoïdes d'agrégats H et/ou J.

3. Utilisation d'agrégats de caroténoïdes selon la revendication 1, **caractérisée par le fait qu'**il s'agit pour les agrégats de caroténoïdes d'agrégats J.

4. Utilisation d'agrégats de caroténoïdes sous la forme H et/ou J pour la coloration de produits alimentaires, de compositions cosmétiques et de compositions pharmaceutiques.

5. Utilisation d'agrégats de caroténoïdes selon la revendication 1 à 4, **caractérisée par le fait que** le caroténoïde est l'astaxanthine ou le lycopène.

6. Boissons, contenant des agrégats de caroténoïdes.

7. Boissons selon la revendication 6, **caractérisées par le fait qu'**elles sont transparentes et contiennent comme caroténoïdes de l'astaxanthine et/ou du lycopène.

8. Boissons selon les revendications 6 et 7, ayant une teneur en caroténoïdes de 0,1 à 100 ppm, par rapport à la quantité totale des compositions.

9. Compositions cosmétiques ou pharmaceutiques, contenant des agrégats de caroténoïdes.

10. Compositions cosmétiques ou pharmaceutiques selon la revendication 9, **caractérisées par le fait qu'**elles contiennent comme caroténoïdes de l'astaxanthine et/ou du lycopène.

11. Compositions cosmétiques ou pharmaceutiques selon les revendications 9 et 10, **caractérisées par le fait qu'**il s'agit alors de compositions transparentes.

12. Compositions cosmétiques ou pharmaceutiques selon les revendications 9 à 11, avec une teneur en caroténoïdes de 0,1 à 100 ppm, par rapport à la quantité totale des compositions.
